# EUROPÄISCHE PATENTSCHRIFT

(11) **EP 2 229 154 B1**
(45) Veröffentlichungstag und Bekanntmachung des Hinweises auf die Patenterteilung: **15.08.2012**
(21) Anmeldenummer: 08858159.0
(22) Anmeldetag: 05.12.2008
(51) Int. Cl.: A61K 9/36, A61K 31/573, A61K 31/565, A61K 31/4439, A61P 15/08

(54) **ARZNEIMITTEL ENTHALTEND EINE KONTRAZEPTIV WIRKENDE HORMONKOMBINATION UND EINEN INSULINSENSITIZER**
PHARMACEUTICAL CONTAINING A HORMONE COMBINATION HAVING A CONTRACEPTIVE EFFECT AND AN INSULIN SENSITIZER
MEDICAMENT CONTENANT UNE COMBINAISON D'HORMONES A EFFET CONTRACEPTIF ET UN AGENT SENSIBILISATEUR A L'INSULINE

(30) Priorität: 05.12.2007 DE 102007058842
(43) Veröffentlichungstag der Anmeldung: 22.09.2010
(73) Patentinhaber: Richter Gedeon Nyrt., 1103 Budapest (HU)
(72) Erfinder: SCHRAMM, Georg, 52224 Stolberg (DE)
(74) Vertreter: Kutzenberger, Helga
(86) Internationale Anmeldenummer: PCT/EP2008/010328
(87) Internationale Veröffentlichungsnummer: WO 2009/071308

(56) Entgegenhaltungen:
- WO-A-02/080971
- WO-A-2005/115350
- WO-A-2007/137797
- WO-A-2008/098318
- CIBULA D ET AL: "The effect of combination therapy with metformin and combined oral contraceptives (COC) versus COC alone on insulin sensitivity, hyperandrogenaemia, SHBG and lipids in PCOS patients" HUMAN REPRODUCTION 200501 GB, Bd. 20, Nr. 1, Januar 2005 (2005-01), Seiten 180-184, XP002521721 ISSN: 0268-1161
- ELTER KORAY ET AL: "Clinical, endocrine and metabolic effects of metformin added to ethinyl estradiol-cyproterone acetate in non-obese women with polycystic ovarian syndrome: A randomized controlled study" HUMAN REPRODUCTION (OXFORD), Bd. 17, Nr. 7, Juli 2002 (2002-07), Seiten 1729-1737, XP002521722 ISSN: 0268-1161
- VRBIKOVA J ET AL: "Combined oral contraceptives in the treatment of polycystic ovary syndrome" HUMAN REPRODUCTION UPDATE, OXFORD UNIVERSITY PRESS, OXFORD, GB, Bd. 11, Nr. 3, 1. Mai 2005 (2005-05-01), Seiten 277-291, XP002449859 ISSN: 1355-4786
- LEMAY A ET AL: "Rosiglitazone and ethinyl estradiol/cyproterone acetate as single and combined treatment of overweight women with polycystic ovary syndrome and insulin resistance" HUMAN REPRODUCTION, OXFORD UNIVERSITY PRESS, GB, Bd. 21, Nr. 1, 1. Januar 2006 (2006-01-01), Seiten 121-128, XP002449856 ISSN: 0268-1161

## Beschreibung

Die vorliegende Erfindung betrifft ein Arzneimittel in Form von Tageseinheiten, die eine Wirkstoffkombination gemäβ Anspruch 1 enthält , und den Einsatz dieser Darreichungsform zur Prophylaxe gegen oder zur Therapierung von krankhaften Störungen verursacht durch das Polyzystische Ovar-Syndrom (PCOS) und zur gleichzeitigen Kontrazeption.

Das Polyzystische Ovar-Syndrom (PCOS) ist mit einer Prävalenz von 5 bis 10% eine der häufigsten Erkrankungen junger Frauen. Symptome dieser Erkrankung sind neben polyzystischen Ovarien mit fortschreitender Erkrankung Oligo- und Amenhorröe, Androgenisierungserscheinungen, wie Hirsutismus, Akne, Alopezie und/oder Adipositas. Als weitere Folgen dieser Erkrankung wird eine zunehmende Insulinresistenz und damit die Gefahr einer Erkrankung an Typ II Diabetes mellitus eine ovulatorische Dysfunktion bis hin zur Unfruchtbarkeit, ein stark erhöhtes Risiko zur Erkrankung an Endometrium-Karziriom, Endometrium-Hyperplasie, Eierstock-Krebs und/oder Brustkrebs sowie an schwerwiegenden kardiovaskulären Erkrankungen, insbesondere ein stark erhöhtes Risiko für Herzinfarkt beobachtet.

Frauen, die an PCOS leiden, bleibt ohne medikamentöse Behandlung ein Kinderwunsch in den meisten Fällen verwehrt, da sowohl durch die unregelmäßigen Ovulationen als auch durch die veränderten endokrinen Parameter, wie beispielsweise erhöhte Blutzuckeniverte, eine (meist reversible) Unfruchtbarkeit einhergeht. PCOS wird üblicherweise mit Hilfe von Insulinsensitizem wie beispielsweise Metformin oder Glitazonen therapiert, welche jedoch zum Teil erhebliche Nebenwirkungen aufweisen und beispielsweise Laktatazidose, kardiovaskuläre Schäden, Leberschäden bzw. embryonale Schäden verursachen können. Daher werden Frauen bei einer Therapierung des PCOS mit Insulinsensitizem üblicherweise noch zusätzlich Kontrazeptiva verabreicht, da im Falle einer (ungewollten) Schwangerschaft das Risiko von Schädigungen des Fötus während der Embryonalentwicklung erhöht ist.

D. Cibula et al. vergleichen den Effekt einer Kombinationstherapie mit Metformin kombiniert mit oralen Kontrazeptiva auf die Insulinsensitivität, die Hyperandrogenaemie, das Sexualhormonbindende Globulin und Lipide bei PCOS Patienten gegenüber einer Monotherapie mit oralen Kontrazeptiva (Human Reproduction Vol. 20, No. 1 pp. 180-184, 2005).

Aufgabe der vorliegenden Erfindung war es daher, ein Arzneimittel bzw. eine Darreichungsform zur Verfügung zu stellen, das zur Prophylaxe gegen oder zur Therapierung von durch das Polyzystische Ovar-Syndrom verursachte krankhafte Störungen und Spätfolgen eingesetzt werden kann, eine Schwangerschaft während einer derartigen Therapierung verhindert, sowie ferner - im Falle eines Kinderwunsches - eine Schwangerschaft mit einem verringerten Risiko von Schädigungen des Fötus während der Embryonalentwicklung nach Beendigung einer Kontrazeption ermöglicht.

Die Aufgabe wird erfindungsgemäß durch das zur Verfügung stellen eines Arzneimittels gelöst, das eine Wirkstoffkombination gemäβ Anspruch 1 enthält.

Die Hormonkombination und wenigstens ein insulinsensitizer sind die einzigen Wirkstoffe des erfindungsgemäßen Arzneimittels.

Unter dem Begriff "Insulinsensitizer" ist im Sinne der vorliegenden Erfindung eine Substanz zu verstehen, welche den Effekten der Insulinresistenz durch eine Erniedrigung der Menge des im Blut zirkulierenden Insulins gegensteuert. Der Insulinsensitizer ist wenigstens eine Verbindung ausgewählt aus der Gruppe bestehend aus Metformin und Glitazonen sowie deren physiologisch verträgliche Salze, wobei das Glitazon vorzugsweise aus der Gruppe bestehend aus Roseglitazon und Pioglitazon ausgewählt ist. Besonders bevorzugt ist der Insulinsensitizer Metformin-Hydrochlorid. Die vorstehend genannten Wirkstoffe sind marktgeführte Produkte.

dIE in dem erfindungsgemäßen Arzneimittel enthaltene, kontrazeptiv wirkende Hormonkombination enthält als Östrogenkomponente wenigstens ein Östrogen ausgewählt aus der Gruppe bestehend aus Estradiol und Ethinylestradiol, wobei natürliches Estradiol besonders bevorzugt ist.

Natürliches Estradiol kann synthetisch hergestellt werden. Entsprechende Synthesen sind bekannt.

Die in dem erfindungsgemäßen Arzneimittel enthaltene, kontrazeptiv wirkende Hormonkombination enthält als Gestagenkomponente wenigstens ein Gestagen ausgewählt aus der Gruppe bestehend aus Chlormadinonacetat, 3-ß-OH-Chlormadinonacetat, 3-α-OH-Chlormadinonacetat, 3-α-Acetoxy-Chlormadinonacetat und 3-β-Acetoxy-Chlormadinonacetat. Die erfindungsgemäß eingesetzte Gestagenkomponente besteht vorzugsweise aus einem einzigen Gestagen oder aus einer Mischung von Gestagenen, die in jedem beliebigen Mischungsverhältnis in der erfindungsgemäßen, kontrazeptiv wirkenden Hormonkombination enthalten sein können. Besonders bevorzugt werden kontrazeptiv wirkende, antiandrogene Gestagene eingesetzt.

In einer bevorzugten Ausführungsform der vorliegenden Erfindung ist die erfindungsgemäß zum Einsatz kommende Gestagenkomponente eine der folgenden Komponenten a) bis h)
a) Chlormadinonacetat oder
b) 3-α-Hydroxy-Chlormadinonacetat oder
c) 3-β-Hydroxy-Chlormadinonacetat oder
d) eine Mischung aus a) und b) oder aus a) und c) oder aus b) und c) oder aus a) und b) und c), jeweils in einem beliebigen Mischungsverhältnis oder
e) 3-α-Acetoxy-Chlormadinonacetat oder
f) 3-β-Acetoxy-Chlormadinonacetat oder
g) eine Mischung aus e) und f) in einem beliebigen Mischungsverhältnis oder
h) eine Mischung aus a) und/oder b) und/oder c) mit e) und/oder f) in einem beliebigen Mischungsverhältnis.

In einer weiteren bevorzugten Ausführungsform der vorliegenden Erfindung ist die erfindungsgemäß zum Einsatz kommende Gestagenkomponente eine der folgenden Komponenten A), B), C) und D)
A) eine Mischung gemäß d), in einem Mischungsverhältnis von 10 bis 90 Gew.-% Chlormadinonacetat (a)) und 90 bis 10 Gew.-% von b) und/oder c), jeweils bezogen auf die Gesamtmischung, oder
B) eine Mischung aus a) und g) in einem Mischungsverhältnis von 10 bis 90 Gew.-% Chlormadinonacetat (a)) und 90 bis 10 Gew.-% von e) und/oder f), jeweils bezogen auf die Gesamtmischung, oder
C) eine Mischung gemäß h), in einem Mischungsverhältnis von 10 bis 90 Gew.-% Chlormadinonacetat (a)) und 90 bis 10 Gew.-% von g), jeweils bezogen auf die Gesamtmischung, oder
D) eine Mischung aus d) und g) in einem Mischungsverhältnis von 10 bis 90 Gew.-% Chlormadinonacetat (a)) und 90 bis 10 Gew.-% von b), c) und g), jeweils bezogen auf die Gesamtmischung.

Das erfindungsgemäße Arzneimittel wird in Form einer bestimmten Anzahl von Tageseinheiten A, bestimmt zur ununterbrochenen, täglichen, oralen Verabreichung an Frauen bereitgestellt.

Um die erfindungsgemäße Darreichungsform zu erhalten, werden die vorstehend genannten Tageseinheiten A mit einer bestimmten Anzahl von Tageseinheiten B kombiniert.

Die erfindungsgemäße Darreichungsform besteht aus einer bestimmten Anzahl von Tageseinheiten A, wie vorstehend beschrieben, bestimmt zur ununterbrochenen, täglichen, oralen Verabreichung an Frauen und aus zusätzlich noch 7-3 hormonfreien, den vorstehend aufgeführten Insulinsensitizer als einzige Wirkstoffkomponente enthaltenden Tageseinheiten B, bestimmt zu einer an die Verabreichung der Tageseinheiten A anschließenden, ununterbrochenen, täglichen, oralen Verabreichung an Frauen.

Bevorzugt liegen die vorstehend genannten Tageseinheiten A und B in Form von Tabletten, vorzugsweise Filmtabletten, vor.

Vorzugsweise enthält jede Tageseinheit A die Gestagenkomponente jeweils wenigstens in einer kontrazeptiv wirksamen Menge bzw. die Östrogenkomponente jeweils wenigstens in einer zyklusstabilisierend wirksamen Menge. Hierbei ist es bevorzugt, dass die Menge des Gestagens mindestens der Ovulationshemmdosis der zum Einsatz kommenden Gestagenkomponente entspricht, die vorzugsweise bis zu 150% über dieser Ovulationshemmdosis liegt. Die entsprechende Ovulationshemmdosis wird in bekannter Weise nach dem Score von "Hoogland & Skouby" bestimmt.

Besonders bevorzugt weist jede Tageseinheit A als Gestagenkomponente eine der vorstehend aufgeführten Komponenten a) bis h) bzw. A) bis D) auf. Besonders bevorzugt wird für die Tageseinheit A Chlormadinonacetat als einzige Gestagenkomponente eingesetzt.

In einer bevorzugten Ausführungsform enthält eine Tageseinheit A Chlormadinonacetat in einer Menge von 1 bis 10 mg, besonders bevorzugt 1 bis 5 mg, ganz besonders bevorzugt jeweils 2, 3, 4 oder 5 mg, 3α-Hydroxy-chlormadinonacetat und/oder 3β-Hydroxy-chlormadinonacetat in einer Menge von 1 bis 20 mg, besonders bevorzugt 1 bis 10, ganz besonders bevorzugt 1 bis 5 mg und Estradiol in einer Menge von 0,1 bis 5 mg, besonders bevorzugt 0,5 bis 3 mg, ganz besonders bevorzugt 1 bis 2 mg oder Ethinylestradiol in einer Menge von 0,001 bis 50 µg, bevorzugter 1 bis 50 µg, besonders bevorzugt 5 bis 30 µg und ganz besonders bevorzugt 20 bis 30 µg, und gegebenenfalls übliche Hilfsstoffe.

Sofern die Tageseinheiten A 3α-Hydroxy-chlormadinonacetat und/oder 3β-Hydroxy-chlormadinonacetat bzw. 3-a-Acetoxy-Chlormadinonacetat und/oder 3-β-Acetoxy-Chlormadinonacetat oder eine Mischung aus wenigstens zwei der vorstehend genannten Komponenten enthalten, beträgt die Menge einheitlich vorzugsweise jeweils 1 bis 20 mg, besonders bevorzugt 1 bis 10 mg, wobei die Estradiol- bzw. Ethinylestradiol-Menge den vorstehend genannten Mengenangaben entspricht.

Vorzugsweise enthält jede Tageseinheit A jeweils dieselbe Menge der zum Einsatz kommenden Gestagenkomponente bzw. dieselbe Menge des zum Einsatz kommenden Estradiols bzw. Ethinylestradiols. Darüber hinaus ist es ebenfalls besonders bevorzugt, dass jede Tageseinheit A dieselbe Gestagenkomponente bzw. dieselbe Östrogenomponente enthält.

Jede Tageseinheit A enthält vorzugsweise dieselbe Menge an Insulinsensitizer, wobei besonders bevorzugt jede Tageseinheit A auch dieselbe Insulinsensitizer-Komponente enthält. Eine Tageseinheit A enthält vorzugsweise 500 bis 2000 mg, besonders bevorzugt 1000 bis 1500 mg Metformin. vorzugsweise als Metforminhydrochlorid, und/oder vorzugsweise 2 bis 45 mg wenigstens eines Glitazons, besonders bevorzugt 5 bis 30 mg Pioglitazon oder besonders bevorzugt 2 bis 8 mg Roseglitazon.

In einer weiteren bevorzugten Ausführungsform der Erfindung enthält jede Tageseinheit A und B denselben Insulinsensitizer und jeweils bis höchstens derselben Menge in einer Tageseinheit A enthaltenen Insulinsensitizer.

Weiterhin enthält jede Tageseinheit B bis höchstens 50 Gew.-%, besonders bevorzugt bis höchstens 40 Gew.-% und nicht weniger als 30 Gew.-% der Menge des in einer Tageseinheit A enthaltenen Insulinsensitizers, wobei es besonders bevorzugt ist, dass die Tageseinheiten A und die Tageseinheiten B denselben Insulinsensitizer und jeweils jede Tageseinheit A bzw. jeweils jede Tageseinheit B dieselbe Menge des Insulinsensitizers enthält.

Dadurch gelingt es, dass - im Falle eines Kinderwunsches von an PCOS-leidenden Frauen - eine Schwangerschaft mit einem verringerten Risiko von Schädigungen des Fötus während der Embryonalentwicklung nach Beendigung einer Kontrazeption bzw. Therapierung ermöglicht wird.

Die Anzahl der Tageseinheiten A und B einer erfindungsgemäßen Darreichungsform kann einem natürlichen, monatlichen, weiblichen Menstruations-Zyklus entsprechen. In diesem Fall weist die erfindungsgemäße Darreichungsform vorzugsweise wenigstens 21-25 Tageseinheiten A und 7-3 Tageseinheiten B, zur ununterbrochenen, oralen, täglichen Einnahme auf. Die Gesamtanzahl der Tageseinheiten A und B beträgt dabei vorzugsweise 28, entsprechend einem natürlichen, weiblichen Monatszyklus.

Es ist aber auch möglich, dass die Gesamtzahl der Tageseinheiten A, welche die erfindungsgemäße Wirkstoffkombination enthalten, größer ist als es einem natürlichen, weiblichen Monatszyklus entspricht, so dass eine erfindungsgemäße Darreichungsform die erfindungsgemäße Wirkstoffkombination enthaltenden Tageseinheiten A für eine ununterbrochene Einnahme bis zu 2 Jahren, vorzugsweise bis zu 1 Jahr, und für eine unmittelbar anschließende ununterbrochene Einnahme 7 bis 3 hormonfreie, Insulinsensitizer-enthaltende Tageseinheiten B aufweisen kann. Es ist aber auch möglich, dass die erfindungsgemäße Darreichungsform 42 bis 52 bzw. 77 bis 193 Tageseinheiten A und zur unmittelbar anschließenden, ununterbrochenen Einnahme 7 bis 3 hormonfreie, den Insulinsensitizer enthaltende Tageseinheiten aufweisen kann.

In einer bevorzugten Ausführungsform der vorliegenden Erfindung wird die erfindungsgemäße Darreichungsform, welche die Tageseinheiten A und B umfasst, in Form von 28, 56, 84, 112, 140, 168, 196, 224, 252, 280, 308, 336 oder 364 Tageseinheiten zur ununterbrochenen oralen, täglichen Verabreichung bereitgestellt. Die erfindungsgemäße Darreichungsform eignet sich zur Prophylaxe gegen oder zur Therapierung von krankhaften Störungen, die durch das Polyzystische Ovar-Syndrom (PCOS) verursacht werden. Besonders bevorzugt eignet sich das erfindungsgemäße Arzneimittel zur Prophylaxe gegen oder zur Therapierung von Insulinresistenz, Oligo- oder Amenhorröe, Hyperandrogenaemie, Typ II Diabetes mellitus (T2 DM), Fettleibigkeit, kardiovaskulären Erkrankungen, Endometrium-Hyperplasie, von weiblicher Unfruchtbarkeit und/oder von androgenen Störungen, wie Hirsutismus, Akne oder/oder Alopezie, zur Prophylaxe gegen Endometrium-Karzinom, Mamma-Karzinom und/oder Eierstock-Krebs, sowie zur gleichzeitigen Kontrazeption.

Ein weiterer Gegenstand der vorliegenden Erfindung ist daher die Verwendung eines Insulinsensitizers zur Herstellung einer Darreichungsform in Form der vorstehend beschriebenen Tageseinheiten A und B, wobei zur Herstellung der Tageseinheiten A neben dem Insulinsensitizer eine der vorstehend beschriebenen, kontrazeptiv wirkenden Hormonkombinationen mitverwendet wird, zur Prophylaxe gegen oder zur Therapierung von krankhaften Störungen, die durch das Polyzystische Ovar-Syndrom (PCOS) verursacht werden. Besonders bevorzugt eignet sich das erfindungsgemäße Arzneimittel zur Prophylaxe gegen oder zur Therapierung von Insulinresistenz, Oligo- oder Amenhorröe, Hyperandrogenaemie, Typ II Diabetes mellitus (T2 DM), Fettleibigkeit, kardiovaskulären Erkrankungen, Endometrium-Hyperplasie, von weiblicher Unfruchtbarkeit und/oder von androgenen Störungen, wie Hirsutismus, Akne oder/oder Alopezie, zur Prophylaxe gegen Endometrium-Karzinom, Mamma-Karzinom und/oder Eierstock-Krebs, sowie zur gleichzeitigen Kontrazeption.

Die erfindungsgemäße Darreichungsform umfassend die Tageseinheiten A und B ist, wie vorstehend beschrieben, vorzugsweise, entsprechend dem weiblichen Menstruationszyklus von 28 Tagen, in einem Blister angeordnet, vorzugsweise unter Kennzeichnung der jeweils einzunehmenden Tageseinheit. Es können jedoch auch mehr als ein Blister mit jeweils 28 Tages-Einheiten, wobei jeder Blister 21-25 Tageseinheiten A und 7-3 Tageseinheiten B aufweist, als Kit zusammengefasst sein. Gegebenenfalls umfasst der Kit auch noch ein Kalender bzw. ein Kalenderbuch.

### Beispiele

### Beispiel 1:

### Zusammensetzung Tageseinheit A

| | Pro Tablette |
|---|---|
| Metformin HCl | 1000 mg |
| Ethinylestradiol | 0,020 mg |
| Chlormadinonacetat | 3 mg |
| Povidon K30 | 40 mg |
| mikrokristalline Cellulose | 200,98 mg |
| Crospovidon | 40 mg |
| Magnesiumstearat | 8 mg |
| Hochdisperses Siliciumdioxid | 8 mg |

Ethinylestradiol (EE) und Povidon K 30 (Polyvinylpyrrolidon, PVP) werden in einer ausreichenden Menge Ethanol gelöst. Chlormadinonacetat (Partikelgröße 90% <50µm), mikrokristalline Cellulose, Metformin HCl und Crospovidon werden in einem Mischer/Granulierer (Diosna P25) 5 min gemischt und anschließend mit der ethanolischen PVP Lösung durchfeuchtet und gemischt. Die feuchte Masse wird durch ein 3 mm Sieb getrieben und in einem Vakuumtrockenschrank getrocknet. Das trockene Granulat wird durch ein 1,0 mm Sieb desagglomeriert, mit Magnesiumstearat und hochdispersem Siliciumdioxid gemischt und zu oblong Tabletten (9 x 21 mm) mit einem Gewicht von 1300 mg gepresst. Die Tabletten werden mit einem Lack auf Basis Methylhydroxypropylcellulose überzogen (z. B. Opadry YS-1-2184); Überzugsmasse 20 mg pro Tablette.

Die Tageseinheiten B wurden auf analoge Weise hergestellt, jedoch ohne Ethinylestradiol und Chlormadinonacetat und mit einer Menge von 204 mg an mikrokristalliner Cellulose. Die Tageseinheiten B enthalten 400 mg Metformin · HCl.

28 dieser Tabletten (21 Tageseinheiten A, 7 Tageseinheiten B) werden zu einer erfindungsgemäßen Darreichungsform in einem Blister mit einer jeweiligen Tagesmarkierung verpackt.

### Beispiel 2:

### Zusammensetzung Tageseinheit A

| | Pro Tablette |
|---|---|
| Pioglitazon | 15 mg |
| Estradiol | 1 mg |
| Chlormadinonacetat | 3 mg |
| Povidon K30 | 5 mg |
| Lactose | 54 mg |
| Maisstärke | 20 mg |
| Magnesiumstearat | 1 mg |
| Hochdisperses Siliciumdioxid | 1 mg |

Estradiol und Povidon K 30 (Polyvinylpyrrolidon, PVP) werden in einer ausreichenden Menge Ethanol gelöst. Chlormadinonacetat (Partikelgröße 90% <50µm), Lactose, Pioglitazon und Maisstärke werden in einem Mischer/Granulierer (Diosna P25) 5 min gemischt und anschließend mit der ethanolischen PVP Lösung durchfeuchtet und gemischt. Die feuchte Masse wird durch ein 3 mm Sieb getrieben und in einem Vakuumtrockenschrank getrocknet. Das trockene Granulat wird durch ein 0,6 mm Sieb desagglomeriert, mit Magnesiumstearat und hochdispersem Siliciumdioxid gemischt und auf einer Tablettenpresse mit 7 mm Stempeln zu Tabletten mit einem Gewicht von 100 mg gepresst.
Die Tabletten wurden mit einem Lack auf Basis Methylhydroxypropylcellulose überzogen (z. B. Opadry YS-1-2184); Überzugsmasse 2 mg pro Tablette

Die Tageseinheiten B werden auf analoge Weise hergestellt, jedoch ohne Mitverwendung von Estradiol und Chlormadinonacetat und mit einer Menge von 65,5 mg an Lactose. Die Tageseinheiten B enthalten 7,5 mg Pioglitazon.

28 dieser Tabletten (21 Tageseinheiten A, 7 Tageseinheiten B) werden zu einer erfindungsgemäßen Darreichungsform in einem Blister mit einer jeweiligen Tagesmarkierung verpackt.

## Patentansprüche

1. Eine Darreichungsform bestehend aus
einem Arzneimittel enthaltend eine Wirkstoffkombination bestehend aus einer kontrazeptiv wirkenden Hormonkombination bestehend aus wenigstens einem Östrogen ausgewählt aus der Gruppe bestehend aus Estradiol und Ethinylestradiol und wenigstens einem Gestagen ausgewählt aus der Gruppe bestehend aus Chlormadinonacetat, 3-ß-OH-Chlormadinonacetat, 3-α-OH-Chlormadinonacetat, 3-α-Acetoxy-Chlormadinonacetat und 3-β-acetoxy-Chlormadinonacetat und wenigstens einen Insulinsensitizer ausgewählt aus der Gruppe bestehend aus Metformin, dessen physiologisch verträgliche Salze und Glitazone in Form einer bestimmten Anzahl von Tageseinheiten A bestimmt zur ununterbrochenen, täglichen, oralen Verabreichung an Frauen,
und zusätzlich noch aus 7-3 hormonfreien, nur den Insulinsensitizer als einzige Wirkstoffkomponente enthaltenden Tageseinheiten B, bestimmt zur daran anschließenden, ununterbrochenen, täglichen, oralen Verabreichung an Frauen,
wobei jede Tageseinheit B bis höchstens 50 Gew.% und nicht weniger als 30 Gew.% der Menge des in einer Tageseinheit A enthaltenen Insulinsensitizers enthält.

2. Eine Darreichungsform nach Anspruch 1, **dadurch gekennzeichnet, dass** das Glitazon ein Glitazon ausgewählt aus der Gruppe bestehend aus Roseglitazon, Pioglitazon und deren physiologisch verträgliche Salze ist.

3. Eine Darreichungsform nach Anspruch 1 oder 2, **dadurch gekennzeichnet, dass** jede Tageseinheit A die Gestagenkomponente jeweils wenigstens in einer kontrazeptiv wirksamen Menge bzw. die Östrogenkomponente jeweils wenigstens in einer zyklusstabilisierend wirksamen Menge enthält.

4. Eine Darreichungsform nach einem der Ansprüche 1 bis 3, **dadurch gekennzeichnet, dass** jede Tageseinheit A dieselbe Menge der Gestagenkomponente bzw. dieselbe Menge des Estradiols oder des Ethinylestradiols enthält.

5. Eine Darreichungsform nach einem der Ansprüche 1 bis 4, **dadurch gekennzeichnet, dass** jede Tageseinheit A dieselbe Gestagenkomponente enthält und sämtliche Tageseinheiten A und B denselben Insulinsensitizer enthalten.

6. Eine Darreichungsform nach einem der Ansprüche 1 bis 5, **dadurch gekennzeichnet, dass** sämtliche Tageseinheiten A jeweils einheitlich Chlormadinonacetat in einer Menge von 1 bis 10 mg oder 3α-Hydroxy-chlormadinonacetat und/oder 3β-Hydroxy-chtormadinonacetat in einer Menge von 1 bis 20 mg und Estradiol jeweils einheitlich in einer Menge von 0,1 bis 5 mg oder Ethinylestradiol jeweils einheitlich in einer Menge von 0,001 bis 50 µg enthalten.

7. Eine Darreichungsform nach einem der Ansprüche 1 bis 6, **dadurch gekennzeichnet, dass** jede Tageseinheit A dieselbe Menge des Insulinsensitizers enthält.

8. Eine Darreichungsform nach einem der Ansprüche 1 bis 7, **dadurch gekennzeichnet, dass** jede Tageseinheit A Metformin in einer Menge von 500 bis 2000 mg und/oder wenigstens ein Glitazon in einer Menge von 2 bis 45 mg enthält.

9. Darreichungsform nach einem der Ansprüche 1 bis 8, **dadurch gekennzeichnet, dass** die Darreichungsform wenigstens 21-25 Tageseinheiten A und 7-3 Tageseinheiten B aufweist.

10. Darreichungsform nach einem der Ansprüche 1 bis 9, **dadurch gekennzeichnet, dass** deren maximale Anzahl an Tageseinheiten A und B für eine ununterbrochene Verabreichung für mehrere Jahre, vorzugsweise für 2 Jahre, besonders bevorzugt für 1 Jahr entspricht.

11. Verwendung wenigstens eines Insulinsensitizers gemäß Anspruch 1 oder 2 zur Herstellung einer Darreichungsform in Form von Tageseinheiten A und B nach wenigstens einem der Ansprüche 1 bis 10, wobei zur Herstellung der Tageseinheiten A neben dem Insulinsensitizer eine kontrazeptiv wirkende Hormonkombination nach wenigstens einem der Ansprüche 3 bis 6 mitverwendet wird, zur Prophylaxe gegen oder Therapierung von durch das Polyzystische Ovar-Syndrom (PCOS) verursachten krankhaften Störungen und zur gleichzeitigen Kontrazeption.

12. Verwendung nach Anspruch 11 zur Prophylaxe gegen oder Therapierung von Insulinresistenz, Oligo- oder Amenhorröe, Hyperandrogenaemie, Fettleibigkeit, Typ II Diabetes mellitus (T2 DM), kardiovaskulären Erkrankungen, Endometrium-Hyperplasie, und zur gleichzeitigen Kontrazeption.

13. Verwendung nach Anspruch 11 zur Prophylaxe gegen Endometrium-Karzinom, Mamma-Karzinom und/oder Eierstock-Krebs oder zur Prophylaxe gegen oder Therapierung von weiblicher Unfruchtbarkeit und zur gleichzeitigen Kontrazeption.

14. Verwendung nach einem der Ansprüche 11 oder 12 zur Prophylaxe gegen oder Therapierung von androgenen Störungen, wie Hirsutismus, Akne oder Alopezie und zur gleichzeitigen Kontrazeption.

## Claims

1. A dosage form consisting of
a medicament containing an active ingredient combination consisting of a hormone combination with contraceptive action consisting of at least one oestrogen selected from the group consisting of estradiol and ethinyl estradiol and at least one gestagen selected from the group consisting of chlormadinone acetate, 3-β-OH-chlormadinone acetate, 3-α-OH-chlormadinone acetate, 3-α-acetoxy-chlormadinone acetate and 3-β-acetoxy-chlormadinone acetate and at least one insulin sensitiser selected from the group consisting of metformin, the physiologically acceptable salts thereof and glitazones in the form of a specific number of daily units A intended for uninterrupted, daily, oral administration to women,
and additionally also of 7-3 hormone-free daily units B containing only the insulin sensitiser as sole active ingredient component, intended for subsequent, uninterrupted, daily, oral administration to women,
each daily unit B containing up to at most 50 wt.% and no less than 30 wt.% of the quantity of the insulin sensitiser contained in a daily unit A.

2. A dosage form according to claim 1, **characterised in that** the glitazone is a glitazone selected from the group consisting of rosiglitazone, pioglitazone and the physiologically acceptable salts thereof.

3. A dosage form according to claim 1 or claim 2, **characterised in that** each daily unit A contains the gestagen component in each case at least in a contraceptively effective quantity respectively the oestrogen component in each case at least in a quantity effective for cycle stabilisation.

4. A dosage form according any one of claims 1 to 3, **characterised in that** each daily unit A contains the same quantity of the gestagen component respectively the same quantity of the estradiol or of the ethinyl estradiol.

5. A dosage form according to any one of claims 1 to 4, **characterised in that** each daily unit A contains the same gestagen component and all the daily units A contain the same insulin sensitiser.

6. A dosage form according to any one of claims 1 to 5, **characterised in that** all the daily units A in each case uniformly contain chlormadinone acetate in a quantity of 1 to 10 mg or 3-α-hydroxy-chlormadinone acetate and/or 3-β-hydroxychlormadinone acetate in a quantity of 1 to 20 mg and estradiol in each case uniformly in a quantity of 0.1 to 5 mg or ethinyl estradiol in each case uniformly in a quantity of 0.001 to 50 µg.

7. A dosage form according to any one of claims 1 to 6, **characterised in that** each daily unit A contains the same quantity of the insulin sensitiser.

8. A dosage form according to any one of claims 1 to 7, **characterised in that** each daily unit A contains metformin in a quantity of 500 to 2000 mg and/or at least one glitazone in a quantity of 2 to 45 mg.

9. A dosage form according to any one of claims 1 to 8, **characterised in that** the dosage form comprises at least 21-25 daily units A and 7-3 daily units B.

10. A dosage form according to any one of claims 1 to 9, **characterised in that** its maximum number of daily units A and B corresponds to uninterrupted administration for one or more years, preferably for 2 years, particularly preferably for 1 year.

11. Use of at least one insulin sensitiser according to claim 1 or claim 2 for producing a dosage form in the form of daily units A and B according to at least one of claims 1 to 10, the daily units A being produced by co-using, in addition to the insulin sensitiser, a hormone combination with a contraceptive action according to at least one of claims 3 to 6, for preventing or treating pathological disorders which are caused by polycystic ovary syndrome (PCOS) and for simultaneous contraception.

12. Use according to claim 11 for preventing or treating insulin resistance, oligomenorrhoea or amenorrhoea, hyperandrogenaemia, obesity, type II diabetes mellitus (T2 DM), cardiovascular diseases, endometrial hyperplasia and for simultaneous contraception.

13. Use according to claim 11 for preventing endometrial carcinoma, mammary carcinoma and/or ovarian cancer or for preventing or treating female infertility and for simultaneous contraception.

14. Use according to either one of claim 11 or claim 12 for preventing or treating androgenic disorders, such as hirsutism, acne or alopecia and for simultaneous contraception.

## Revendications

1. Forme d'administration, constituée par
un médicament contenant une combinaison de substances actives, constituée par une combinaison hormonale à effet contraceptif, constituée par au moins un estrogène choisi dans le groupe formé par l'estradiol et l'éthinylestradiol et au moins un gestagène choisi dans le groupe formé par l'acétate de chlormadinone, l'acétate de 3-ß-OH-chlormadinone, l'acétate de 3-α-OH-chlormadinone, l'acétate de 3-α-acétoxy-chlormadinone et l'acétate de 3-ß-acétoxy-chlormadinone et au moins un agent insulinosensibilisant, choisi dans le groupe formé par la metformine, ses sels physiologiquement acceptables et les glitazones sous forme d'un nombre déterminé d'unités journalières A, destinées à une administration ininterrompue, journalière, par voie orale à des femmes,
et en plus encore par 7-3 unités journalières B exemptes d'hormones, ne contenant que l'agent insulinosensibilisant comme unique composant de substance active, destinées à une administration consécutive, ininterrompue, journalière par voie orale à des femmes,
chaque unité journalière B contenant au maximum jusqu'à 50% en poids et pas moins de 30% en poids de la quantité de l'agent insulinosensibilisant contenu dans une unité journalière A.

2. Forme d'administration selon la revendication 1, **caractérisée en ce que** la glitazone est une glitazone choisie dans le groupe formé par la rosiglitazone, la pioglitazone et leurs sels physiologiquement acceptables.

3. Forme d'administration selon la revendication 1 ou 2, **caractérisée en ce que** chaque unité journalière A contient le composant gestagène à chaque fois au moins en une quantité contraceptivement active ou, selon le cas, le composant estrogène à chaque fois au moins en une quantité active pour stabiliser le cycle.

4. Forme d'administration selon l'une quelconque des revendications 1 à 3, **caractérisée en ce que** chaque unité journalière A contient la même quantité de composant gestagène ou, selon le cas, la même quantité d'estradiol ou d'éthinylestradiol.

5. Forme d'administration selon l'une quelconque des revendications 1 à 4, **caractérisée en ce que** chaque unité journalière A contient le même composant gestagène et l'ensemble des unités journalières A et B contiennent le même agent insulinosensibilisant.

6. Forme d'administration selon l'une quelconque des revendications 1 à 5, **caractérisée en ce que** l'ensemble des unités journalières A contiennent à chaque fois de manière unique de l'acétate de chlormadinone en une quantité de 1 à 10 mg ou de l'acétate de 3-α-hydroxychlormadinone et/ou de l'acétate de 3-ß-hydroxychlormadinone en une quantité de 1 à 20 mg et l'estradiol à chaque fois de manière unique en une quantité de 0,1 à 5 mg ou l'éthinylestradiol à chaque fois de manière unique en une quantité de 0,001 à 50 µg.

7. Forme d'administration selon l'une quelconque des revendications 1 à 6, **caractérisée en ce que** chaque unité journalière A contient la même quantité d'agent insulinosensibilisant.

8. Forme d'administration selon l'une quelconque des revendications 1 à 7, **caractérisée en ce que** chaque unité journalière A contient de la metformine en une quantité de 500 à 2000 mg et/ou au moins une glitazone en une quantité de 2 à 45 mg.

9. Forme d'administration selon l'une quelconque des revendications 1 à 8, **caractérisée en ce que** la forme d'administration présente au moins 21-25 unités journalières A et 7-3 unités journalières B.

10. Forme d'administration selon l'une quelconque des revendications 1 à 9, **caractérisée en ce que** son nombre maximal d'unités journalières A et B correspond à une administration ininterrompue pour plusieurs années, de préférence 2 années, en particulier 1 année.

11. Utilisation d'au moins un agent insulinosensibilisant selon la revendication 1 ou 2 pour la préparation d'une forme d'administration sous forme d'unités journalières A et B selon au moins l'une quelconque des revendications 1 à 10, en utilisant conjointement, pour la préparation des unités journalières A, outre l'agent insulinosensibilisant, une combinaison hormonale à effet contraceptif selon au moins l'une quelconque des revendications 3 à 6, pour la prophylaxie ou la thérapie de troubles maladifs provoqués par le syndrome des ovaires polykystiques (SOPK) et simultanément pour la contraception.

12. Utilisation selon la revendication 11 pour la prophylaxie ou la thérapie de l'insulinorésistance, de l'oligoménorrhée ou de l'aménorrhée, de l'hyperandrogénisme, de l'obésité, du diabète sucré de type II (DS T2), des maladies cardiovasculaires, de l'hyperplasie de l'endomètre et simultanément pour la contraception.

13. Utilisation selon la revendication 11 pour la prophylaxie du carcinome de l'endomètre, du carcinome mammaire et/ou du cancer des ovaires ou pour la prophylaxie ou la thérapie de l'infertilité féminine et simultanément pour la contraception.

14. Utilisation selon l'une quelconque des revendications 11 ou 12 pour la prophylaxie ou la thérapie de troubles androgènes, tels que l'hirsutisme, l'acné ou l'alopécie et simultanément pour la contraception.
